Europäisches Patentamt

(19) European Patent Office

Office européen des brevets

(11) **EP 0 993 826 B1**

(12) **FASCICULE DE BREVET EUROPEEN**

(45) Date de publication et mention
de la délivrance du brevet:
**04.07.2001 Bulletin 2001/27**

(51) Int Cl.[7]: **A61K 7/48**, A61K 7/06,
A61K 7/42

(21) Numéro de dépôt: **99401905.7**

(22) Date de dépôt: **26.07.1999**

(54) **Utilisation d' un extrait de Sanguisorba officinalis pour favoriser la pigmentation de la peau et/ou des cheveux**

Verwendung von Sanguisorba officinalis Extrakten zur Förderung der Haut- und/oder Haar-Pigmentierung

Use of a Sanguisorba officinalis extract to stimulate the skin- and/or the hair-pigmentation

(84) Etats contractants désignés:
**AT BE CH CY DE DK ES FI FR GB GR IE IT LI LU MC NL PT SE**

(30) Priorité: **07.09.1998 FR 9811154**

(43) Date de publication de la demande:
**19.04.2000 Bulletin 2000/16**

(73) Titulaire: **L'OREAL**
**75008 Paris (FR)**

(72) Inventeurs:
• **Pelletier, Pascale**
**92160 Antony (FR)**

• **Regnier, Marcelle**
**75018 Paris (FR)**

(74) Mandataire: **Renard, Emmanuelle**
**L'OREAL/DPI**
**6, Rue Bertrand Sincholle**
**92585 Clichy Cedex (FR)**

(56) Documents cités:
**EP-A- 0 375 082        EP-A- 0 727 217**

• **PATENT ABSTRACTS OF JAPAN vol. 013, no. 137 (C-582) & JP 63 303910 A (KANEBO)**

**Description**

La présente invention se rapporte à l'utilisation d'au moins un extrait d'une rosacée du genre *Sanguisorba* dans une composition cosmétique et/ou pour la fabrication d'une composition dermatologique, l'extrait et/ou la composition étant destiné à favoriser la pigmentation de la peau et/ou des poils et/ou des cheveux, ainsi qu'à l'utilisation d'au moins un extrait d'une rosacée du genre *Sanguisorba* comme agent propigmentant et/ou stimulateur de la mélanogénèse dans une composition cosmétique et/ou pour la fabrication d'une composition dermatologique. La présente invention concerne également un procédé cosmétique de pigmentation de la peau et/ou des poils et/ou des cheveux consistant à appliquer sur la peau et/ou les poils et/ou les cheveux une composition comprenant au moins un extrait d'une rosacée du genre *Sanguisorba.*

La couleur des cheveux et de la peau humaine est fonction de différents facteurs et notamment des saisons de l'année, de la race, du sexe et de l'âge. Elle est principalement déterminée par la concentration dans les kératinocytes de la mélanine produite par les mélanocytes. Les mélanocytes sont les cellules spécialisées qui, par l'intermédiaire d'organelles particuliers, les mélanosomes, synthétisent la mélanine.

La synthèse de la mélanine ou mélanogénèse est particulièrement complexe et fait intervenir schématiquement les principales étapes suivantes :

Tyrosine ---> Dopa ---> Dopaquinone ---> Dopachrome ---> Mélanine

La tyrosinase (monophénol dihydroxyl phénylalanine : oxygen oxydoreductase / EC 1,14,18,1) est l'enzyme essentielle intervenant dans cette suite de réactions. Elle catalyse notamment la réaction de transformation de la tyrosine en Dopa (dihydroxyphénylalanine) et la réaction de transformation de la Dopa en Dopaquinone.

Dans l'épiderme, le mélanocyte est impliqué dans l'unité mélanique épidermique qui comporte un mélanocyte entouré d'environ 36 kératinocytes voisins. Tous les individus, sans distinction de phototype, ont approximativement le même nombre de mélanocytes pour une zone cutanée donnée. Les différences ethniques, en terme de pigmentation, ne sont pas dues au nombre de mélanocytes, mais aux propriétés de leurs mélanosomes. Les mélanosomes sont agrégés en complexes et sont de petites tailles. Ce sont des organelles hautement spécialisées dont l'unique fonction est la production de mélanine. Ils naissent du réticulum endoplasmique sous formes de vacuoles sphériques appelées prémélanosomes. Les prémélanosomes contiennent un substrat protéinique amorphe, mais pas d'enzymes mélanogènes. Au cours de la maturation du prémélanosome, le substrat amorphe s'organise en une structure fibrillaire orientée dans l'axe longitudinal du mélanosome. On distingue quatre stades du développement du mélanosome correspondant à l'intensité de la mélanisation. La mélanine est déposée uniformément sur le réseau fibrillaire interne du mélanosome et l'opacité de l'organelle augmente jusqu'à saturation. Au fur et à mesure que la mélanine est synthétisée dans les mélanosomes, ceux-ci se déplacent de la région périnucléaire vers l'extrémité des dendrites des mélanocytes. Par phagocytose, l'extrémité des dendrites est capturée par les kératinocytes, les membranes dégradées et les mélanosomes redistribués dans les kératinocytes.

Bien que le taux de mélanine varie d'une population à une autre, la quantité de tyrosinase ne varie pas significativement et le taux d'ARN messagers de la tyrosinase est identique dans des peaux blanches ou noires. Les variations dans la mélanogénèse sont donc dues à des variations soit de l'activité de la tyrosinase soit de la capacité des kératinocytes à phagocyter les mélanosomes.

On sait que, dans la plupart des populations, l'obtention d'une coloration brune de la peau et le maintien d'une coloration constante de la chevelure sont des aspirations importantes.

Il existe par ailleurs des maladies de la pigmentation comme par exemple le vitiligo qui est une maladie autoimmune se caractérisant par l'apparition sur la peau de plaques blanches liées à un défaut de pigmentation.

Il existe donc un réel besoin de produit facilitant et/ou améliorant la pigmentation de la peau et/ou des poils et/ou des cheveux.

De nombreuses solutions ont été proposées dans le domaine de la coloration artificielle par apport de colorants exogènes, tels que la DHA, censés donner à la peau et/ou aux poils et/ou aux cheveux une coloration la plus proche possible de ce qu'elle est naturellement ou, dans le domaine de la coloration naturelle, par stimulation des voies naturelles de pigmentation, par exemple en utilisant des actifs stimulant la mélanogénèse avec ou sans action des UV, comme l'-αMSH ou les prostaglandines.

Par exemple, il a été proposé dans les documents WO-A-9517161, WO-9511003, WO-A-9501773, WO-A-9404674, WO-A-9404122, EP-A-585018, WO-A-9310804, WO-A-9220322 ou WO-A-9107945 des solutions aussi variées que des compositions contenant un inhibiteur de phosphodiestérases, l'utilisation de prostaglandines, de fragments d'ADN ou encore de dérivés de la tyrosine.

D'excellents résultats sont certes obtenus par les solutions proposées dans l'art antérieur, mais les composés utilisés présentent souvent des effets secondaires non négligeables ou sont des mélanges complexes qui ne présentent pas de spécificité.

La découverte de substances ayant un effet sur la pigmentation de la peau et/ou des poils et/ou des cheveux sans présenter d'effets secondaires gênants reste un objectif majeur de la recherche.

**EP 0 993 826 B1**

La demanderesse a maintenant découvert que, de façon surprenante, un extrait d'une rosacée du genre *Sanguisorba,* de préférence de l'espèce *Sanguisorba officinalis*, avait un effet activateur de la mélanogénèse, même à faible concentration, sans faire preuve de cytotoxicité. Cet extrait permet d'obtenir une coloration naturelle et en profondeur de la peau et/ou des poils et/ou des cheveux, ainsi qu'une protection vis-à-vis des UV, contrairement aux colorants de type exogène.

*Sanguisorba officinalis* est une plante herbacée des régions tempérées, encore appelée Grande Pimprenelle, qui appartient à la famille des rosacées. La racine de *Sanguisorba officinalis* est essentiellement constituée de composés phénoliques et de triterpène glycosides. Parmi les composés phénoliques, on peut citer l'acide caféique et l'acide chlorogénique, des coumarines simples et des furocoumarines, mais surtout des tannins et en particulier des ellagitannins, ainsi que des catéchols et des gallo-catéchols. *Sanguisorba officinalis* contient également des acides organiques (acides quinique et tiglique) et une huile essentielle contenant des terpénoïdes.

La composition de la racine de *Sanguisorba officinalis* justifie son utilisation en médecine traditionnelle chinoise comme hémostatique, antiseptique et anti-inflammatoire, en particulier contre les hémorragies hémorroïdaires et utérines, et comme astringent dans le traitement des brûlures et de l'eczéma. Il a également été proposé d'utiliser *Sanguisorba officinalis* pour ses propriétés anti-radicalaires et anti-oxydantes, dans des préparations cosmétiques anti-âge.

D'autres compositions pour application topique sur la peau sont également décrites dans EP-A-0 375 082, JP-A-03258711 et JP-63 303 910.

Précisément, EP-A-0 375 082 divulgue une composition renfermant un extrait de rhizome de *Sanguisorba officinalis et* un phosphatide de type monoacyle, utilisable sous forme de crème contre l'acné ou de lotion pour les cheveux, par exemple. Dans le document de brevet japonais JP-A-03258711, il est fait référence à l'utilisation d'un extrait de racines de *Sanguisorba officinalis* pour l'obtention d'un pentagalloylglucose destiné à éclaircir la peau. Une autre composition éclaircissante contenant un extrait de *Sanguisorba officinalis* est divulguée dans le document de brevet japonais JP-63 303 910, dans lequel de la saponine extraite de *Sanguisorba officinalis* est associée à de la vitamine E.

Toutefois, à ce jour, à la connaissance de la Demanderesse; il n'a encore jamais été proposé d'utiliser un extrait de rosacée du genre *Sanguisorba* pour favoriser la pigmentation de la peau et/ou des poils et/ou des cheveux et/ou comme propigmentant.

L'invention a donc pour objet l'utilisation d'au moins un extrait de rosacée du genre *Sanguisorba* dans une composition cosmétique et/ou pour la fabrication d'une composition dermatologique, l'extrait et/ou la composition étant destiné à favoriser la pigmentation de la peau et/ou des poils et/ou des cheveux.

L'invention a également pour objet l'utilisation d'au moins un extrait de rosacée du genre *Sanguisorba* comme agent propigmentant et/ou stimulateur de la mélanogénèse dans une composition cosmétique et/ou pour la fabrication d'une composition dermatologique.

L'invention a encore pour objet un procédé cosmétique de pigmentation de la peau et/ou des poils et/ou des cheveux consistant à appliquer sur la peau et/ou les poils et/ou les cheveux une composition comprenant au moins un extrait de rosacée du genre *Sanguisorba.*

L'extrait utilisé dans l'invention est avantageusement un extrait de *Sanguisorba officinalis*, de préférence préparé à partir de racines et/ou de rhizome de cette espèce.

Toute méthode d'extraction connue de l'homme du métier peut être utilisée pour préparer cet extrait. On peut en particulier citer les extraits aqueux, alcooliques ou utilisant un solvant organique.

Par solvant aqueux on entend tout solvant constitué totalement ou pour part d'eau. On peut citer ainsi l'eau elle-même, les solvants hydroalcooliques en toute proportion ou encore les solvants constitués d'eau et d'un composé comme le propylène glycol en toute proportion.

Parmi les solvants alcooliques on peut citer notamment l'éthanol.

Quel que soit le mode de préparation utilisé selon l'invention, des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélangée dans un solvant approprié avant utilisation.

Préférentiellement, selon l'invention, on utilise un extrait sec de racine et de rhizome de *Sanguisorba officinalis* qui peut être obtenu sous forme de poudre auprès de MARUZEN PHARMACEUTICALS CO., LTD., Japon.

La quantité d'extrait contenue dans la composition selon l'invention est bien évidemment fonction de l'effet recherché et pourra de ce fait varier dans une large mesure. D'une manière générale, l'extrait de rosacée du genre *Sanguisorba* sera donc présent dans la composition en une quantité efficace pour obtenir le degré de pigmentation voulu et avantageusement en une quantité supérieure à 0,002% du poids total de la composition, par exemple en une quantité comprise entre environ 0,002 et environ 10% en poids, mieux, en une quantité comprise entre environ 0,1% et environ 5% en poids et, encore mieux, en une quantité d'environ 0,5% en poids.

La composition selon l'invention est destinée à être appliquée sur la peau et/ou les poils et/ou les cheveux en vue de favoriser leur pigmentation et/ou de stimuler la mélanogénèse et, à cette fin, elle peut se présenter sous toute forme galénique convenant à une application topique.

La composition de l'invention peut ainsi se présenter sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que des nanosphères et des nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les poils ou sur les cheveux sous forme d'aérosol contenant également un agent propulseur sous pression. Elle peut également se présenter sous forme solide, et par exemple sous forme de stick. Elle peut être utilisée comme produit de soin et/ou comme produit de maquillage. Elle peut en variante se présenter sous la forme d'un shampooing ou d'un après-shampooing.

De façon connue, la composition de l'invention peut contenir également les adjuvants habituels dans les domaines cosmétique et dermatologique, tels que des gélifiants hydrophiles ou lipophiles, des actifs hydrophiles ou lipophiles, des conservateurs, des antioxydants, des solvants, des parfums, des charges, des filtres, des pigments, des absorbeurs d'odeur et des matières colorantes. Les quantités de ces différents adjuvants sont celles classiquement utilisées dans les domaines considérés, et ils représentent par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (Carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols (glycérine, propylène glycol), les vitamines, les agents kératolytiques et/ou desquamants (acide salicylique et ses dérivés, alpha-hydroxyacides, acide ascorbique et ses dérivés), les agents anti-inflammatoires, les agents apaisants et leurs mélanges.

L'invention a également pour objet un procédé de traitement cosmétique pour augmenter la pigmentation de la peau et/ou des poils et/ou des cheveux et/ou stimuler la mélanogénèse, consistant à appliquer sur la peau et/ou les poils et/ou les cheveux une composition cosmétique comprenant au moins un extrait de rosacée du genre *Sanguisorba* dans un milieu cosmétiquement acceptable.

Par milieu cosmétiquement acceptable, on entend un milieu compatible avec la peau, les muqueuses, les ongles, les poils, les cheveux.

L'invention sera mieux comprise, et ses avantages ressortiront mieux, à la lumière des exemples non limitatifs suivants, donnés à titre d'illustration seulement.

Exemple 1 : Mesure de l'effet modulateur de la mélanogénèse d'un extrait de *Sanguisorba officinalis* - Co-culture de kératinocytes et mélanocytes

1.1. MATERIEL ET METHODE

L'effet modulateur sur la mélanogénèse d'un extrait pulvérulent de *Sanguisorba officinalis* obtenu auprès de MARUZEN PHARMACEUTICALS CO., LTD., Japon a été testé selon la méthode décrite dans le brevet FR-A-2 734 825 déposé par la Demanderesse, ainsi que dans l'article de R. Schmidt, P. Krien et M. Régnier, Anal. Biochem., 235 (2), 113-18, 1996.

En bref, la méthode comprend les étapes consistant à :

- mettre en co-culture des kératinocytes / mélanocytes humains normaux dans un milieu contenant l'extrait testé ainsi qu'un précurseur de mélanine marqué, en l'occurrence du thiouracile marqué au $C^{14}$,
- à lyser les cellules contenant la mélanine avec des enzymes dégradant les protéines,
- à faire passer l'extrait obtenu sur un filtre échangeur d'anions ayant un diamètre de pores inférieur ou égal à 1 μm, et
- à évaluer la quantité de mélanine fixée par le filtre au moyen d'un dosage radioactif du thiouracile.

Précisément, 250 000 kératinocytes humains normaux et 80 000 mélanocytes humains normaux sont mélangés et ensemencés par puits de plaques de 24 puits (type Costar) et cultivés trois jours dans le milieu de différenciation. Pendant les trois jours suivants, le milieu de culture est remplacé quotidiennement par le milieu test défini (contenant dans un solvant l'extrait selon l'invention, ci-après « Extrait S.O. », aux concentrations de 0,001%, 0,002%, 0,005% et 0,1%, respectivement, ainsi que 1 μCi/ml de thiouracile marquée au $C^{14}$).

Les témoins suivants sont réalisés :

- culture témoin : pas d'extrait à tester ;
- témoin positif de stimulation de la mélanogénèse : 1 mM tyrosine ;
- témoin positif d'inhibition de la mélanogénèse : 500 μM d'acide kojique.

La radioactivité totale incorporée dans les protéines est estimée par l'incorporation de leucine tritiée, prise comme indicateur de cytotoxicité et de prolifération. La veille de la prise, 1 μCi/ml de leucine tritiée est ajouté au milieu test.

Après une nuit, les cellules sont rincées en tampon phosphate. Les protéines sont précipitées à l'aide d'acide trichloracétique (TCA) à 5% et lavées afin d'éliminer la radioactivité libre. Les protéines sont incubées pendant la nuit à 40°C à l'aide d'une solution de protéinase K à 100 μg/ml dans du tampon Tris HCl-triton-EDTA.

50 μl d'extrait total sont prélevés et transférés dans une plaque 24 puits (Wallac) et 500 μl de liquide scintillant (Optiphase « Supermix ») sont ajoutés. Le reste de l'extrait, soit 950 μl, est filtré sur filtre DEAE Filtermat. Après rinçage, le filtre recouvert du scintillant solide « Meltilex » est transféré dans une plaque. La radioactivité est comptée à l'aide du compteur Wallac. Les résultats sont exprimés en pourcentage du témoin selon la formule:

$$\frac{(14CP / 3HP) - (14CT / 3HT)}{(14CT / 3HT)} \times 100$$

dans laquelle:

$14CP$ est la moyenne des désintégrations par minute (dpm) thiouracile $^{14}C$ sur 3 puits similaires traités par un produit (P) ;
$3HP$ est la moyenne des dpm leucine $^{3}H$ correspondant ;
$14CP$ est la moyenne des désintégrations par minute (dpm) thiouracile $^{14}C$ sur 3 puits similaires traités par un produit (P) ;
$3HP$ est la moyenne des dpm leucine $^{3}H$ correspondant ;
$14CT$ est la moyenne des dpm thiouracile $^{14}C$ sur 3 puits similaires témoin (T) ;
$3HT$ est la moyenne des dpm leucine $^{3}H$ correspondant.

On calcule le rapport de l'incorporation de thiouracile à l'incorporation de leucine, qui traduit la stimulation de la mélanogénèse. Les produits testés sont ensuite classés en fonction de leur activité selon la nomenclature suivante :

0 à 30% de stimulation de la synthèse de mélanine correspond à un propigmentant de classe 1
30 à 60% de stimulation de la synthèse de mélanine correspond à un propigmentant de classe 2
60 à 100% de stimulation de la synthèse de mélanine correspond à un propigmentant de classe 3
plus de 100% de stimulation de la synthèse de mélanine correspond à un propigmentant de classe 4.

1.2. RESULTATS

| Produits | $^3$H Leu. (%/Témoin) | $^{14}$C ThioU. (%/Témoin) | $\frac{^{14}C\ ThioU.}{^3H\ Leu.}$ |
|---|---|---|---|
| Tyrosine 1 mM | -3 | 42 | 46 |
| Solvant (éthanol) 0,1% | 21 | -6 | -23 |
| Extrait S.O. 0,1% | -12 | 54 | 114 |
| Acide kojique 500 µM | 11 | -18 | -26 |

| Produits | $^3$H Leu. (%/Témoin) | $^{14}$C ThioU. (%/Témoin) | $\frac{^{14}C\ ThioU.}{^3H\ Leu.}$ |
|---|---|---|---|
| Tyrosine 1 mM | 1 | 76 | 74 |
| Solvant (DMSO) 0,1% | -5 | 16 | 23. |
| Extrait S.O. 0,001% | -5 | -3 | 1 |
| Extrait S.O. 0,002% | 4 | 79 | 72 |
| Extrait S.O. 0,005% | -9 | 635 | 705 |
| Acide kojique 500 µM | -9 | -39 | -32 |

1.3. CONCLUSIONS

Ce produit est non cytotoxique aux concentrations testées (variation non significative de l'incorporation de $^3$H Leu.) et induit la synthèse de mélanine à partir de 0,002% (augmentation de l'incorporation de $^{14}$CThioU).
Le rapport de l'incorporation de thiouracile à l'incorporation de leucine traduit la stimulation de la mélanogénèse, qui peut atteindre 700 fois la valeur du témoin, correspondant à un propigmentant de classe 4.
La concentration activatrice de 50% de la pigmentation, ou $AC_{50}$, est comprise entre 0,002% et 0,005%.

Exemple 2 : Mesure de l'effet modulateur de la mélanogénèse de l'extrait de *Sanguisorba officinalis* - Essai sur épiderme reconstruit pigmenté.

2.1. MATERIEL ET METHODES

Cet essai a été réalisé en utilisant un modèle d'épiderme reconstruit pigmenté tel que décrit dans Journal of Investigative Dermatology, Vol. 102, 1994 par M. Régnier et R. Schmidt. Ce modèle présente l'avantage d'être plus proche des conditions in vivo. On applique exactement le même mode opératoire que dans l'Exemple 1, en détachant l'épiderme avant la lyse des cellules.
Les cellules utilisées sont les kératinocytes et les mélanocytes. Après cinq jours d'immersion et sept jours d'émersion, l'épiderme reconstruit pigmenté est traité avec l'extrait de l'Exemple 1 utilisé à la concentration de 0,01% dans le DMSO. 3 µl de solution sont appliqués chaque jour pendant cinq jours, deux nouvelles applications étant effectuées après l'interruption du week-end Les échantillons sont prélevés à seize jours d'émersion finale pour l'histologie (coloration à l'hématoxiline-éosine et au Fontana-Masson), les macrophotographies et la mesure de colorimétrie au Microflash.
Un lambeau d'épiderme reconstruit est détaché du support et traité pour évaluer son effet activateur sur l'activité dopa-oxydase de la tyrosinase des mélanocytes humains. L'épiderme est mis en présence d'un co-substrat réducteur (la L-dopa en petites quantités) pour initier la réaction d'hydroxylation de la L-tyrosine en L-dopa, laquelle est ensuite oxydée catalytiquement en dopaquinone puis en dopachrome, produit intermédiaire avant les réactions d'oxydation non enzymatiques aboutissant à la formation de mélanine. La réaction est lue au microscope optique.

2.2. RESULTATS

2.2.1. Colorimétrie

On utilise la classification de A. CHARDON (CHARDON et al., in Biological Responses to UVA Radiation, Ed. F. Urbach, 1992, Valdenmach Publ. Co. Overland Park, KS) permettant de classer les épidermes de « peaux très claires » à « peaux très bronzées » en fonction des angles typologiques individuels. L'épiderme reconstruit traité se situe dans la classe des « peaux bronzées », tandis qùe l'épiderme reçonstruit témoin (traité avec le DMSO) se situe dans la classe des « peaux intermédiaires ».

2.2.2. Activité tyrosinase

La mise en évidence, sur lambeau d'épiderme reconstruit détaché, de l'activité tyrosinase révélée à l'aide de la réaction DOPA, montre une augmentation de l'activité dans les cultures traitées.

2.2.3. Quantité de mélanine

L'évaluation de la quantité de mélanine sur coupes histologiques colorées au Fontana-Masson montre une augmentation de la quantité de pigment dans les cultures traitées.

2.3. CONCLUSIONS

Ces résultats confirment l'effet fortement propigmentant de l'extrait de *Sanguisorba officinalis* selon l'invention.

Exemple 3 : Exemple de composition contenant au moins un extrait de *Sanguisorba officinalis*

Cette composition peut être obtenue par les techniques habituelles couramment utilisées en cosmétique ou en pharmacie. Les pourcentages indiqués sont des pourcentages pondéraux.

| Poudre d'extrait de *Sanguisorba officinalis* | | 0,5% |
|---|---|---|
| Glycérine | | 7,0% |
| Alcools | | 5,5% |
| Carbomer | | 0,3% |
| Hydroxyde de sodium | | 0,1% |
| Conservateurs | | 0,6% |
| Eau | qsp | 100,0% |

La composition est un gel propigmentant.

**Revendications**

1. Utilisation non-thérapeutique d'au moins un extrait de rosacée du genre *Sanguisorba* dans une composition cosmétique, l'extrait et/ou la composition étant destiné à favoriser la pigmentation de la peau et/ou des poils et/ou des cheveux.

2. Utilisation d'au moins un extrait de rosacée du genre *Sanguisorba* pour la fabrication d'une composition dermatologique, l'extrait et/ou la composition étant destiné à favoriser la pigmentation de la peau et/ou des poils et/ou des cheveux.

3. Utilisation non-thérapeutique d'au moins un extrait de rosacée du genre *Sanguisorba* comme agent propigmentant et/ou stimulateur de la mélanogénèse dans une composition cosmétique.

4. Utilisation d'au moins un extrait de rosacée du genre *Sanguisorba* comme agent propigmentant et/ou stimulateur de la mélanogénèse pour la fabrication d'une composition dermatologique.

5. Utilisation selon l'une quelconque des revendications 1 à 4, caractérisée en ce que ledit extrait est présent en une quantité comprise entre 0,002% et 10% en poids, par rapport au poids total de ladite composition.

6. Utilisation selon la revendication 5, caractérisée en ce que ledit extrait est présent en une quantité comprise entre 0,1% et 5% en poids, par rapport au poids total de ladite composition.

7. Utilisation selon la revendication 6, caractérisée en ce que ledit extrait est présent en une quantité d'environ 0,5% en poids, par rapport au poids total de ladite composition.

8. Utilisation selon l'une quelconque des revendications 1 à 7, caractérisée en ce que ledit extrait est un extrait de *Sanguisorba officinalis*.

9. Utilisation selon la revendication 8, caractérisée en ce que ledit extrait est un extrait de racine et/ou de rhizome de *Sanguisorba officinalis*.

10. Procédé cosmétique de pigmentation de la peau et/ou des poils et/ou des cheveux consistant à appliquer sur la peau et/ou les poils et/ou les cheveux une composition cosmétique comprenant une quantité efficace d'au moins un extrait de rosacée du genre *Sanguisorba* dans un milieu cosmétiquement acceptable.

**Patentansprüche**

1. Nicht therapeutische Verwendung mindestens eines Extrakts von Rosaceae der Gattung *Sanguisorba* in einer kosmetischen Zusammensetzung, wobei der Extrakt und/oder die Zusammensetzung dazu vorgesehen sind, die Pigmentierung der Haut und/oder der Körperhaare und/oder der Haare zu fördern.

2. Verwendung mindestens eines Extrakts von Rosaceae der Gattung *Sanguisorba* zur Herstellung einer dermatologischen Zusammensetzung, wobei der Extrakt und/oder die Zusammensetzung dazu vorgesehen sind, die Pigmentierung der Haut und/oder der Körperhaare und/oder der Haare zu fördern.

3. Nicht therapeutische Verwendung mindestens eines Extrakts von Rosaceae der Gattung *Sanguisorba* als pigmentierungsförderndes Mittel und/oder als Stimulator der Melanogenese in einer kosmetischen Zusammensetzung.

4. Verwendung mindestens eines Extrakts von Rosaceae der Gattung *Sanguisorba* als pigmentierungsförderndes Mittel und/oder als Stimulator der Melanogenese zur Herstellung einer dermatologischen Zusammensetzung.

5. Verwendung nach einem der Ansprüche 1 bis 4, dadurch gekennzeichnet, daß der Extrakt in einem Mengenanteil von 0,002 bis 10 Gew.-%, bezogen auf das Gesamtgewicht der Zusammensetzung, vorliegt.

6. Verwendung nach Anspruch 5, dadurch gekennzeichnet, daß der Extrakt in einem Mengenanteil von 0,1 bis 5 Gew.-%, bezogen auf das Gesamtgewicht der Zusammensetzung, vorliegt.

7. Verwendung nach Anspruch 6, dadurch gekennzeichnet, daß der Extrakt in einem Mengenanteil von etwa 0,5 Gew.-%, bezogen auf das Gesamtgewicht der Zusammensetzung, vorliegt.

8. Verwendung nach einem der Ansprüche 1 bis 7, dadurch gekennzeichnet, daß es sich bei dem Extrakt um einen Extrakt von *Sanguisorba officinalis* handelt.

9. Verwendung nach Anspruch 8, dadurch gekennzeichnet, daß der Extrakt ein Wurzelextrakt und/oder Rhizom-Extrakt von *Sanguisorba officinalis* ist.

10. Kosmetisches Verfahren zur Pigmentierung der Haut und/oder der Körperhaare und/oder der Haare, das darin besteht, auf die Haut und/oder die Körperhaare und/oder die Haare eine kosmetische Zusammensetzung aufzutragen, die in einem kosmetisch akzeptablen Medium mindestens einen Extrakt von Rosaceae der Gattung *Sanguisorba* in einer wirksamen Menge enthält.

**Claims**

1. Non-therapeutic use of at least one extract of a rosaceous plant of the genus *Sanguisorba* in a cosmetic composition, with the extract and/or the composition being intended for promoting pigmentation of the skin and/or the body hair and/or the cranial hair.

2. Use of at least one extract of a rosaceous plant of the genus *Sanguisorba* for producing a dermatological composition, with the extract and/or the composition being intended for promoting pigmentation of the skin and/or the body hair and/or the cranial hair,

3. Non-therapeutic use of at least one extract of a rosaceous plant of the genus *Sanguisorba* as a propigmentation agent and/or stimulator of melanogenesis in a cosmetic composition.

4. Use of at least one extract of a rosaceous plant of the genus *Sanguisorba* as a propigmentation agent and/or stimulator of melanogenesis for producing a dermatological composition.

5. Use according to any one of Claims 1 to 4, characterized in that the said extract is present in a quantity of between 0.002% and 10% by weight, based on the total weight of the said composition.

6. Use according to Claim 5, characterized in that the said extract is present in a quantity of between 0.1% and 5% by weight, based on the total weight of the said composition.

7. Use according to Claim 6, characterized in that the said extract is present in a quantity of approximately 0.5% by weight, based on the total weight of the said composition.

8. Use according to any one of Claims 1 to 7, characterized in that the said extract is an extract of *Sanguisorba officinalis*.

9. Use according to Claim 8, characterized in that the said extract is an extract of the root and/or the rhizome of *Sanguisorba officinalis*.

10. Cosmetic process for pigmenting the skin and/or the body hair and/or the cranial hair, which process consists in applying, to the skin and/or the body hair and/or the cranial hair, a cosmetic composition which comprises an effective quantity of at least one extract of a rosaceous plant of the genus *Sanguisorba* in a cosmetically acceptable medium.